# EUROPÄISCHE PATENTANMELDUNG

(11) **EP 3 640 631 A1**
(43) Veröffentlichungstag der Anmeldung: **22.04.2020**
(21) Anmeldenummer: 19203294.4
(22) Anmeldetag: 15.10.2019
(51) Int. Cl.: G01N 25/20, G01N 33/00

(54) **THERMISCHER GASSENSOR, VERFAHREN ZUR MESSUNG DER TEMPERATURLEITFÄHIGKEIT EINES GASES ODER GASGEMISCHS UND VERFAHREN ZUR MESSUNG DER WÄRMELEITFÄHIGKEIT EINES GASES ODER GASGEMISCHS**

(30) Priorität: 19.10.2018 DE 102018008286
(71) Anmelder: Diehl Metering GmbH, 91522 Ansbach (DE)
(72) Erfinder: Hammerschmidt, Ulf, 38112 Braunschweig (DE); Benkert, Andreas, 91522 Ansbach (DE); Sosna, Christoph, 90429 Nürnberg (DE); Herrmann, Karl, 90542 Eckental (DE)
(74) Vertreter: Diehl Patentabteilung

(57) **Zusammenfassung**

Thermischer Gassensor zur Messung der Temperaturleitfähigkeit und/oder der Wärmeleitfähigkeit eines Gases oder Gasgemischs, umfassend ein Substrat (2), in dessen Oberfläche (3) ein Graben (4) ausgebildet ist, sowie wenigstens zwei beabstandet voneinander auf der Oberfläche (3) des Substrates (4) angeordnete Leiterstrukturen (7, 8), wobei die Leiterstrukturen (7, 8) jeweils wenigstens zwei Kontaktabschnitte (9) sowie einen mit diesen Kontaktabschnitten (9) verbundenen Stegabschnitt (10) umfassen, wobei die Stegabschnitte (10) der Leiterstrukturen (7, 8) beabstandet voneinander den Graben (4) überspannen, wobei in wenigstens einem Bereich der Oberfläche (3) des Substrates (2) zwischen wenigstens zwei Kontaktabschnitten (9) unterschiedlicher Leiterstrukturen (7, 8) wenigstens ein Schlitz (11) ausgebildet ist.

## Beschreibung

Die Erfindung betrifft einen thermischen Gassensor zur Messung der Temperaturleitfähigkeit und/oder der Wärmeleitfähigkeit eines Gases oder Gasgemischs, umfassend ein Substrat, in dessen Oberfläche ein Graben ausgebildet ist, sowie wenigstens zwei beabstandet voneinander auf der Oberfläche des Substrates angeordnete Leiterstrukturen, wobei die Leiterstrukturen jeweils wenigstens zwei Kontaktabschnitte sowie einen mit diesen Kontaktabschnitten verbundenen Stegabschnitt umfassen, wobei die Stegabschnitte der Leiterstrukturen beabstandet voneinander den Graben überspannen. Weiterhin betrifft die Erfindung ein Verfahren zur Messung der Temperaturleitfähigkeit eines Gases oder Gasgemischs sowie ein Verfahren zur Messung der Wärmeleitfähigkeit eines Gases oder Gasgemischs.

Ein derartiger thermischer Gassensor kann beispielsweise zur Bestimmung der Temperaturleitfähigkeit von Erdgasen eingesetzt werden. Dazu wird zwischen den Leiterstrukturen ein durch das zu untersuchende Gas oder Gasgemisch strömender Wärmefluss erzeugt. Über die zur Wärmeausbreitung zwischen den Leiterstrukturen erforderliche Zeit kann auf die Temperaturleitfähigkeit des Gases oder Gasgemisches zurückgeschlossen werden. Parasitärer Wärmefluss, das heißt Wärmeströme, die sich nicht über das zu untersuchende Gas ausbreiten, sondern beispielsweise über das Substrat fließen, stellen dabei ein Problem dar. Derartige Wärmeströme verfälschen das Messergebnis beziehungsweise machen aufwändige Anpassungen der zur Bestimmung der Temperaturleitfähigkeit des Gases verwendeten mathematischen Modelle erforderlich. Analog trifft dies auch bei der Messung einer Wärmeleitfähigkeit eines Gases oder eines Gasgemischs mithilfe eines derartigen thermischen Gassensors zu,
Der Erfindung liegt daher die Aufgabe zu Grunde, einen verbesserten thermischen Gassensor anzugeben, der parasitäre Wärmeströme vermeidet oder reduziert.

Zur Lösung dieser Aufgabe ist erfindungsgemäß vorgesehen, dass in wenigstens einem Bereich der Oberfläche des Substrates zwischen wenigstens zwei Kontaktabschnitten unterschiedlicher Leiterstrukturen wenigstens ein Schlitz ausgebildet ist.

Durch den zwischen den Kontaktabschnitten unterschiedlicher Leiterstrukturen vorgesehenen Schlitz wird die zwischen den Kontaktabschnitten bestehende Festkörperbrücke über das Substrat des thermischen Gassensors zumindest teilweise unterbrochen. Dies bietet den Vorteil, dass ein durch das Substrat fließender, parasitärer Wärmefluss zwischen den wenigstens zwei Leiterstrukturen vermieden oder zumindest reduziert wird. Durch die Vermeidung bzw. die Reduktion dieses parasitären Wärmeflusses ist eine genauere und einfachere Bestimmung der Temperaturleitfähigkeit und/oder der Wärmeleitfähigkeit des Gases oder Gasgemischs durch den thermischen Gassensor möglich.

Die wenigstens zwei Kontaktabschnitte der Leiterstrukturen befinden sich insbesondere zu beiden Seiten des Grabens und sind mit dem Stegabschnitt der Leiterstruktur verbunden, der den Graben insbesondere freitragend überspannt. Die wenigstens zwei Leiterstrukturen des thermischen Gassensors sind derart auf der Oberfläche des Substrates angeordnet, dass ihre Stegabschnitte beabstandet zueinander den Graben überspannen. Zur Messung der Temperaturleitfähigkeit eines die Stegabschnitte umströmenden Gases bzw. Gasgemischs wird eine thermische Laufzeit eines Wärmeflusses zwischen einem als Heizelement betriebenen Stegabschnitt einer Leiterstruktur sowie einem als Temperaturfühler betriebenen Stegabschnitt einer weiteren Leiterstruktur gemessen. Je besser das die Stegabschnitte umgebende Gas die Wärme transportiert, desto schneller erreicht der von dem Heizelement erzeugte Temperaturanstieg den Temperaturfühler. Die Zeit bzw. Laufzeit, bis die Temperatur des Temperaturfühlers auf ihr Maximum ansteigt, bildet ein Maß für die Temperaturleitfähigkeit des Gases. Die Leiterstrukturen mit den als Temperaturfühler verwendeten Stegelementen können beispielsweise als Widerstandsthermometer betrieben werden.

Zwischen den Kontaktabschnitten zweier benachbarter Leiterstrukturen kann insbesondere jeweils wenigstens ein Schlitz vorgesehen sein, um in dem Bereich der Oberfläche des Substrates zwischen den wenigstens zwei Kontaktabschnitten der benachbarten Leiterstrukturen den parasitären Wärmefluss über das Substrat zu reduzieren oder zu unterbinden. Insbesondere kann es vorgesehen sein, dass zwischen mehreren oder allen Kontaktabschnitten eines Teils oder aller Leiterstrukturen zwischen den jeweils benachbarten Kontaktabschnitten ein oder mehrere Schlitze vorgesehen sind, so dass sich insgesamt nur ein geringer und insbesondere zu vernachlässigender Wärmefluss über das Substrat ergibt.

Erfindungsgemäß kann vorgesehen sein, dass sich der Graben entlang einer Längsrichtung erstreckt, wobei die Stegabschnitte der Leiterstrukturen den Graben parallel oder im Wesentlichen parallel in einer Querrichtung überspannen. Die Stegabschnitte der Leiterstrukturen sind folglich entlang der Längsrichtung versetzt voneinander angeordnet. Zu einer Leiterstruktur mit einem als Heizelement betriebenen Stegabschnitt können weitere Leiterstrukturen derart angeordnet werden, dass ihre Stegabschnitte in unterschiedlichen Abständen zu dem Heizelement den Graben überspannen. Es ist auch möglich, dass bei mehreren weiteren Leiterstrukturen diese bezogen auf die Längsrichtung der Ausdehnung des Grabens vor und hinter dem als Heizelement eingesetzten Stegelement angeordnet sind.

Für den Graben kann erfindungsgemäß vorgesehen sein, dass er einen Boden, der insbesondere parallel oder im Wesentlichen parallel zur Oberfläche des Substrates ist, sowie wenigstens zwei Wände umfasst, wobei die Wände zwischen der Oberfläche des Substrates und dem Boden des Grabens verlaufen, wobei sich der wenigstens eine Schlitz wenigstens von der Oberseite des Substrates zum Boden des Grabens erstreckt und/oder wobei der wenigstens eine Schlitz durch eine Wand des Grabens verläuft. Der Graben kann einen quadratischen, rechteckigen oder trapezförmigen Querschnitt aufweisen. Der wenigstens eine Schlitz kann sich von dem Graben durch den Bereich der Oberfläche des Substrates zwischen den zwei Kontaktabschnitten benachbarter Leiterstrukturen erstrecken. Selbstverständlich ist es möglich, dass bei Kontaktstrukturen, die zu beiden Seiten des Grabens auf der Oberfläche des Substrates angeordnet sind, auch durch beide Wände des Grabens jeweils wenigstens ein Schlitz geführt ist. Der Schlitz kann zumindest abschnittsweise orthogonal zu der Wand des Grabens beziehungsweise zu der Verlaufsrichtung des Grabens verlaufen.

Erfindungsgemäß kann vorgesehen sein, dass der Schlitz einen oder mehrere aneinander anschließende gerade Abschnitte umfasst, wobei der Schlitz den Bereich der Oberfläche des Substrates zwischen den wenigstens zwei Kontaktabschnitten ganz oder teilweise durchschneidet. Dabei kann vorgesehen sein, dass der Schlitz zumindest abschnittsweise der Kontur beziehungsweise der Kantengeometrie eines oder beider der Kontaktabschnitte folgt. Bevorzugt verläuft der Schlitz im Wesentlichen mittig zwischen zwei Kontaktabschnitten benachbarter Leiterstrukturen. Mehrere aneinander anschließende gerade Abschnitte eines Schlitzes können unter verschiedenen Winkeln aneinander anschließen, so dass der Schlitz in Abhängigkeit einer Form der Kontaktabschnitte in verschiedenen und gegebenenfalls abschnittweise an die Form einer oder mehrerer benachbarter Kontaktabschnitte angepassten Geometrien verlaufen kann.

Zur Fertigung des Grabens und/oder des Schlitzes kann erfindungsgemäß vorgesehen sein, dass der Graben und/oder der wenigstens einen Schlitz durch ein bereichsweises Ätzen des Substrates gefertigt ist. Das Ätzen kann dabei beispielsweise als nasschemisches Ätzen und/oder als Trockenätzen erfolgen.

Erfindungsgemäß kann vorgesehen sein, dass der Schlitz eine Breite zwischen 1 µm und 50 µm, insbesondere zwischen 10 µm und 20 µm, aufweist und/oder dass der Graben eine Breite zwischen 0,5 mm und 5 mm und/oder eine Tiefe zwischen 100 µm und 500 µm aufweist. Die Breite des Schlitzes ist dabei unabhängig von der Breite und/oder der Tiefe des Grabens. Es kann sich für den Fertigungsprozess als vorteilhaft erweisen, wenn der wenigstens eine Schlitz dieselbe Tiefe aufweist wie der Graben, jedoch ist es auch möglich, dass der Schlitz tiefer oder weniger tief als der Graben ist und/oder dass sich bei mehreren vorhandenen Schlitzen ihre Tiefen und/oder ihre Breiten unterscheiden.

In einer bevorzugten Ausgestaltung der Erfindung kann vorgesehen sein, dass die Leiterstrukturen über die wenigstens zwei Kontaktabschnitte zur Bestromung des Stegabschnitts der Leiterstruktur bestrombar und/oder zur Messung eines Widerstandes des Stegabschnitts kontaktierbar sind, wobei wenigstens eine der Leiterstrukturen zwei weitere Kontaktabschnitte zur Messung eines Potentialabfalls über dem Stegabschnitt aufweist. Für eine Leiterstruktur, deren Stegabschnitt im Betrieb des thermischen Gassensors als Heizelement dient, sind zwei Kontaktabschnitte zur Bestromung des Stegabschnittes ausreichend. Bei den weiteren Leiterstrukturen, die im Betrieb des thermischen Gassensors beispielsweise als Widerstandsthermometer eingesetzt werden, sind grundsätzlich ebenfalls zwei Kontaktabschnitte zur Messung des Widerstandes des Stegabschnittes ausreichend. Für eine genaue Bestimmung des Widerstandes eines Stegabschnittes einer als Widerstandsthermometer eingesetzten Leiterstruktur kann es zu Kalibrierungszwecken jedoch vorteilhaft sein, wenn diese Leiterstrukturen vier Kontaktabschnitte aufweisen, von denen jeweils zwei auf einer Seite des Grabens auf der Oberfläche des Substrates angeordnet sind. Das Vorsehen von vier Kontaktabschnitten an diesen Leiterstrukturen ermöglicht es, zur Bestimmung des Widerstandes des Stegabschnitts eine Vierpunktmessung an den Leiterstrukturen durchzuführen. Für den Betrieb des thermischen Gassensors, also zur Messung der Temperaturleitfähigkeit und/oder der Wärmeleitfähigkeit eines die Stegabschnitte umströmenden Gases oder Gasgemisches ist es jedoch ausreichend, wenn eine Widerstandsmessung des Stegabschnitts über zwei Kontakte durchgeführt wird. Um die Leiterstrukturen zu bestromen, beziehungsweise um die elektrischen Widerstände der Leiterstrukturen und insbesondere der Stegabschnitte zu messen, können Kontaktmittel wie beispielsweise Messnadeln und/oder Bondkontakte auf die Kontaktabschnitte der Leiterstrukturen aufgebracht werden, um den Anschluss des thermischen Gassensors an eine zur Ansteuerung und zur Messung einsetzbare Steuerungseinrichtung und/oder an entsprechende Messgeräte vorzunehmen.

Erfindungsgemäß kann vorgesehen sein, dass auf der Oberfläche des Substrates zu zwei gegenüberliegenden Seiten einer ein Heizelement bildenden Leiterstruktur mit zwei Kontaktabschnitten jeweils eine ein Widerstandsthermometer bildende Leiterstruktur mit vier Kontaktabschnitten angeordnet ist, wobei zwischen den Kontaktabschnitten des Heizelements und jeweils wenigstens einem Kontaktabschnitt der Widerstandsthermometer ein Schlitz ausgebildet ist. Eine Messung kann auf diese Weise über eine Wärmeausbreitung in zwei entgegengesetzt verlaufenden Richtungen der Längsrichtung des Grabens vorgenommen werden. Zusätzlich zu den Schlitzen zwischen den Kontaktabschnitten der ein Heizelement bildenden Leiterstruktur sowie der benachbart angeordneten, jeweils ein Widerstandsthermometer bildenden Leiterstrukturen können selbstverständlich auch zwischen weiteren, jeweils als Widerstandsthermometer eingesetzten Leiterstrukturen Schlitze zwischen den jeweiligen Kontaktabschnitten vorgesehen sein.

Erfindungsgemäß kann vorgesehen sein, dass die Leiterstrukturen zumindest teilweise aus Nickel bestehen und/oder dass das Substrat zumindest teilweise aus Silizium und/oder Siliziumnitrid besteht. Eine Nickelschicht, aus der die Leiterstrukturen gebildet sind, kann dabei beispielsweise zwischen 50 nm und 300 nm, insbesondere ca. 200 nm, dick sein. Als Substrat kann beispielsweise Silizium verwendet werden, welches an der Oberfläche mit Siliziumnitrid beschichtet ist, auf dem die Leiterstrukturen aufgebracht sind.

Durch die Siliziumnitridschicht wird eine isolierende Oberfläche des Substrates geschaffen, so dass zwischen den jeweiligen Leiterstrukturen kein oder nur ein vernachlässigbar geringer Stromfluss auftritt.

Für ein erfindungsgemäßes Verfahren zur Messung der Temperaturleitfähigkeit eines Gases oder Gasgemisches mittels eines erfindungsgemäßen thermischen Gassensors ist vorgesehen, dass es die Schritte umfasst:
- Bereitstellen des thermischen Gassensors sowie des Gases oder des Gasgemischs,
- Anlegen eines zeitlich begrenzten Strompulses an eine Leiterstruktur,
- zeitabhängige Messung des elektrischen Widerstandes wenigstens einer weiteren Leiterstruktur,
- Bestimmung der Temperaturleitfähigkeit aus der zeitabhängigen Messung des elektrischen Widerstandes der wenigstens einen weiteren Leiterstruktur und dem Abstand der Stegabschnitte der Leiterstrukturen zueinander.

Der thermische Gassensor kann dazu beispielsweise mit einer zur Durchführung des Verfahrens ausgebildeten Messeinrichtung, zum Beispiel einem Mikrokontroller oder Ähnlichem, verbunden sein. Selbstverständlich ist es möglich, dass mehr als eine Leiterstruktur als Widerstandsthermometer eingesetzt wird, wobei sich die Stegabschnitte dieser Leiterstrukturen insbesondere in verschiedenen Abständen und/oder in verschiedenen Richtungen von dem als Heizelement eingesetzten Stegabschnitt befinden können.

Für ein erfindungsgemäßes Verfahren zur Messung der Wärmeleitfähigkeit eines Gases oder Gasgemisches mittels eines erfindungsgemäßen thermischen Gassensors, welcher wenigstens drei beabstandet voneinander auf der Oberfläche des Substrates angeordnete Leiterstrukturen umfasst, ist vorgesehen, dass es die Schritte umfasst:
- Bereitstellen des thermischen Gassensors sowie des Gases oder Gasgemischs,
- Anlegen eines zeitlich begrenzten Strompulses an eine Leiterstruktur zum Heizen dieser Leiterstruktur,
- Messung von Temperaturwerten mittels der wenigstens zwei weiteren Leiterstrukturen,
- Bestimmung der Wärmeleitfähigkeit aus Temperaturwerten der wenigstens zwei weiteren Leiterstrukturen sowie dem Abstand der Stegabschnitte der zwei weiteren Leiterstrukturen zu dem Stegabschnitt der geheizten Leiterstruktur.

Der thermische Gassensor kann dazu beispielsweise mit einer zur Durchführung des Verfahrens ausgebildeten Messeinrichtung, zum Beispiel einem Mikrokontroller oder Ähnlichem, verbunden sein. Selbstverständlich ist es möglich, dass mehr als zwei weitere Leiterstrukturen als Widerstandsthermometer eingesetzt werden, wobei sich die Stegabschnitte dieser Leiterstrukturen insbesondere in verschiedenen Abständen und/oder in verschiedenen Richtungen von dem als Heizelement eingesetzten Stegabschnitt befinden können. Die wenigstens zwei weiteren Leiterstrukturen können als Widerstandsthermometer betrieben werden, wobei als Temperaturwert der Widerstand des Stegabschnitts, welcher proportional zu der Temperatur des Stegabschnitts verwendet wird, herangezogen wird. Zusätzlich oder alternativ dazu kann als Temperaturwert auch ein Strom durch den Stegabschnitt bei konstanter Spannung oder eine über dem Stegabschnitt abfallende Spannung bei konstantem Strom herangezogen werden. Auch die Temperatur des als Heizelement betriebenen Stegabschnitts kann über eine entsprechende Widerstandsmessung bestimmt werden.

Aus den Temperaturwerten der weiteren Leiterstrukturen können anschließend Parameter berechnet werden, die eine Ausgleichsgerade parametrisieren, welche den Zusammenhang zwischen dem Logarithmus der Entfernung des jeweiligen Stegabschnitts einer weiteren Leiterstruktur von dem Stegabschnitt der geheizten Leiterstruktur und dem Temperaturmesswert beschreibt. Aus den Parametern kann anschließend die Wärmeleitfähigkeit des Gases oder Gasgemischs bestimmt werden. Aufgrund des wenigstens einen Schlitzes zwischen wenigstens zwei der Leiterstrukturen wird parasitärer Wärmefluss über das Substrat reduziert oder unterbunden, so dass eine genauere Bestimmung der Wärmeleitfähigkeit des Gases oder Gasgemischs möglich ist.

Die Verwendung eines Strompulses zum Heizen hat den Vorteil, dass eine konstante Erwärmung weiterer Strukturen des Gassensors, wie beispielsweise des Substrates, vermieden wird, wodurch die Wärmeleitfähigkeit genauer bestimmt werden kann. Für die Bestimmung der Wärmeleitfähigkeit wird auf die deutsche Patentanmeldung DE 10 2018 006 868.5 verwiesen, in welcher die Bestimmung der Wärmeleitfähigkeit aus den Parametern genauer erläutert wird.

Weitere Vorteile und Einzelheiten der Erfindung ergeben sich aus den im Folgenden beschriebenen Ausführungsbeispielen sowie anhand der Zeichnungen. Dabei zeigen:
- Fig. 1: eine schematische Aufsicht eines ersten Ausführungsbeispiels eines erfindungsgemäßen thermischen Gassensors,
- Fig. 2: eine perspektivische Ansicht des ersten Ausführungsbeispiels des erfindungsgemäßen thermischen Gassensors,
- Fig. 3: eine schematische Aufsicht auf ein zweites Ausführungsbeispiel eines erfindungsgemäßen thermischen Gassensors, und
- Fig. 4: eine Aufsicht auf ein drittes Ausführungsbeispiel eines erfindungsgemäßen thermischen Gassensors.

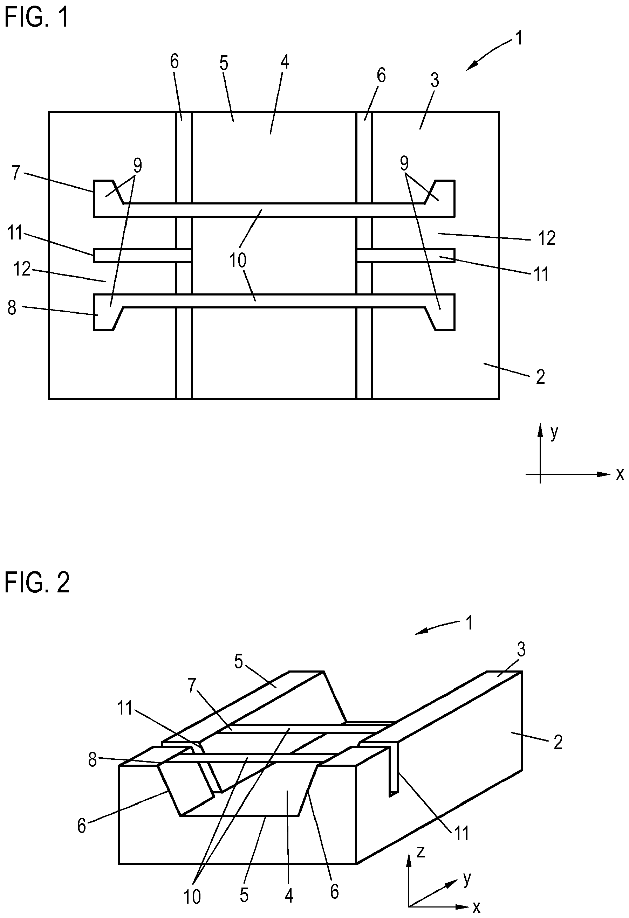

In Fig. 1 ist eine schematische Aufsicht auf ein erstes Ausführungsbeispiel eines erfindungsgemäßen thermischen Gassensors 1 dargestellt. Der Gassensor 1 umfasst ein Substrat 2, in dessen Oberfläche 3 ein Graben 4 ausgebildet ist. Der Graben 4 umfasst einen Boden 5, der im Wesentlichen parallel zu der Oberfläche 3 des Substrates 2 ist, sowie zwei Wände 6, die den Boden 5 mit der Oberfläche 3 des Substrates 2 verbinden. Der Graben 4 weist in diesem Ausführungsbeispiel einen trapezförmigen Querschnitt auf, der aus der perspektivischen Darstellung des ersten Ausführungsbeispiels in Fig. 2 ersichtlich ist. In Fig. 1 weiterhin dargestellt sind zwei Leiterstrukturen 7, 8, die jeweils zwei Kontaktabschnitte 9 sowie einen Stegabschnitt 10 umfassen. Als Stegabschnitt 10 wird dabei der Teil der Leiterstruktur 9 bezeichnet, der den Graben 4 überspannt. Zu beiden Seiten des Grabens schließt der den Graben 4 überspannende Stegabschnitt 10 an jeweils einen Kontaktabschnitt 7 der jeweiligen Leiterstruktur 9 an. Zwischen den benachbarten Kontaktabschnitten 9 der Leiterstrukturen 7, 8 ist auf beiden Seiten des Grabens 7 jeweils ein Schlitz 11 vorgesehen. Dieser Schlitz 11 verläuft in dem Bereich 12 der Oberfläche 3 des Substrates 2 zwischen den jeweiligen Kontaktabschnitten 9 der benachbarten Leiterstrukturen 7, 8.

Die Schlitze 11 erstecken sich dabei jeweils durch eine Wand 6 des Grabens 4 und verlaufen in Querrichtung zu dem Graben 4, hier in positiver beziehungsweise negativer X-Richtung, entsprechend der Ausdehnung der Leiterstrukturen 7, 8. Selbstverständlich ist es möglich, dass die Schlitze 11 in positiver beziehungsweise in negativer X-Richtung eine kleinere oder auch größere Ausdehnung als die Leiterstrukturen 7, 8 aufweisen.

Durch die Schlitze 11 wird zwischen den Kontaktabschnitten 9 der Leiterstrukturen 7, 8 ein parasitärer Wärmefluss durch das Substrat 2 zumindest bereichsweise verhindert oder reduziert. Bei der Messung der Temperaturleitfähigkeit und/oder der Wärmeleitfähigkeit eines die Stegabschnitte 10 des Gassensors 1 umströmenden Gases oder Gasgemisches wird in diesem Ausführungsbeispiel beispielsweise die Leiterstruktur 7 über ihre Kontaktabschnitte 9 zumindest kurzzeitig bestromt, so dass sich der Stegabschnitt 10 der Leiterstruktur 7 erwärmt. Die von dem erwärmten Stegabschnitt 10 der Leiterstruktur 7 abgegebene Wärme breitet sich in Abhängigkeit der Temperaturleitfähigkeit des die Stegabschnitte 10 umströmenden Gases oder Gasgemisches aus, so dass es abhängig von dem Abstand der Stegabschnitte 10 zueinander nach einer gewissen Zeit den Stegabschnitt 10 der Leiterstruktur 8 erreicht und anschließend erwärmt. Die Erwärmung des Stegabschnittes 10 der Leiterstruktur 7 führt zu einer Veränderung des elektrischen Widerstandes des Stegabschnittes 10 der Leiterstruktur 8, wobei die sich dadurch ergebende Widerstandsänderung über die Kontaktabschnitte 9 der Leiterstruktur 8 gemessen werden kann. Durch eine zeitabhängige Messung des Widerstandes des Stegabschnittes 10 der Leiterstruktur 8 kann somit unter Berücksichtigung des Abstandes der Stegabschnitte 10 zueinander auf die Temperaturleitfähigkeit des die Stegabschnitte 10 umgebenden Gases oder Gasgemisches geschlossen werden. Die durch die Schlitze 11 erfolgende Reduktion der parasitären Wärmeflüsse an beiden Seiten des Grabens zwischen den Kontaktabschnitten 9 der Leiterstrukturen 7, 8 verbessert folglich die Funktionalität des Sensors, da eine Wärmeausbreitung von sich nicht über das Gas oder Gasgemisch ausbreitender Wärme zwischen den Leiterstrukturen 7, 8 unterbunden oder reduziert wird, so dass genauer und/oder mit einem einfacheren mathematischen Modell zur Beschreibung der Wärmeausbreitung gemessen werden kann.

In Fig. 2 ist eine schematische perspektivische Ansicht des ersten Ausführungsbeispiels des thermischen Gassensors 1 dargestellt. Der Übersichtlichkeit halber ist nur der Bereich um den Graben 4 dargestellt. Ersichtlich ist der trapezförmige Querschnitt des Grabens 4, der durch den Grabenboden 5 sowie die beiden Wände 6 des Grabens gebildet wird. Die dargestellten Stegabschnitte 10 der Leiterstrukturen 7, 8 überspannen den Graben 4 freitragend und schließen zu beiden Seiten, wie in Fig. 1 gezeigt ist, jeweils an einen in Fig. 2 nicht dargestellten Kontaktabschnitt 9 an.

Die Schlitze 11, die die Oberfläche 3 des Substrates 2 zwischen den Kontaktabschnitten 9 durchschneiden, weisen in diesem Ausführungsbeispiel dieselbe Tiefe wie der Graben 4 auf und durchschneiden jeweils eine Wand 6 des Grabens 4. Der Graben kann dabei eine Tiefe zwischen 100 µm und 500 µm aufweisen. Die Breite der Schlitze in Längsrichtung des Grabens 4, hier also in Y-Richtung, beträgt beispielsweise zwischen 1 µm und 50 µm, insbesondere zwischen 10 µm und 20 µm. Der Graben kann eine Breite in Querrichtung, hier also in X-Richtung, zwischen 0,5 mm und 5 mm aufweisen. Durch die Schlitze 11 wird, wie hier ersichtlich ist, zumindest bereichsweise ein Festkörperkontakt zwischen dem Substrat 2 unterhalb der Leiterstruktur 7 sowie dem Substrat 2 unterhalb der Leiterstruktur 8 unterbrochen, so dass in diesen Bereichen keine oder nur eine reduzierte Wärmeleitung durch das Substrat erfolgen kann. Das Substrat 2 besteht beispielsweise aus Silizium, das an der Oberfläche ganz oder zumindest bereichsweise mit einer Isolationsschicht, beispielsweise aus Siliziumnitrid, beschichtet ist, um einen parasitären Stromfluss zwischen den Leiterstrukturen 7, 8 zu unterbinden beziehungsweise gering zu halten. Die Leiterstrukturen 7, 8 können beispielsweise aus Nickel mit einer Breite, das heißt in Bezug zu Fig. 2 mit einer Ausdehnung in Y-Richtung, zwischen 1 µm und 20 µm, beispielsweise von 10 µm, und einer Dicke, das heißt in Bezug zu Fig. 2 mit einer Ausdehnung in Z-Richtung, zwischen 50 nm und 300 nm, beispielsweise von 200 nm, bestehen.

Für die Schlitze 11 kann vorgesehen sein, dass diese eine andere Tiefe als der Graben 4 aufweisen. Beispielsweise ist es möglich, dass die Schlitze 11 eine geringere und/oder eine größere Tiefe als der Graben 4 aufweisen. Alternativ kann auch vorgesehen sein, dass die Schlitze 11 die Wand 6 des Grabens 4 nicht durchschneiden, wobei am Rand des Grabens 4 im Bereich der Wand 6 jeweils ein kleiner Verbindungssteg des Substrates 2 verbleibt.

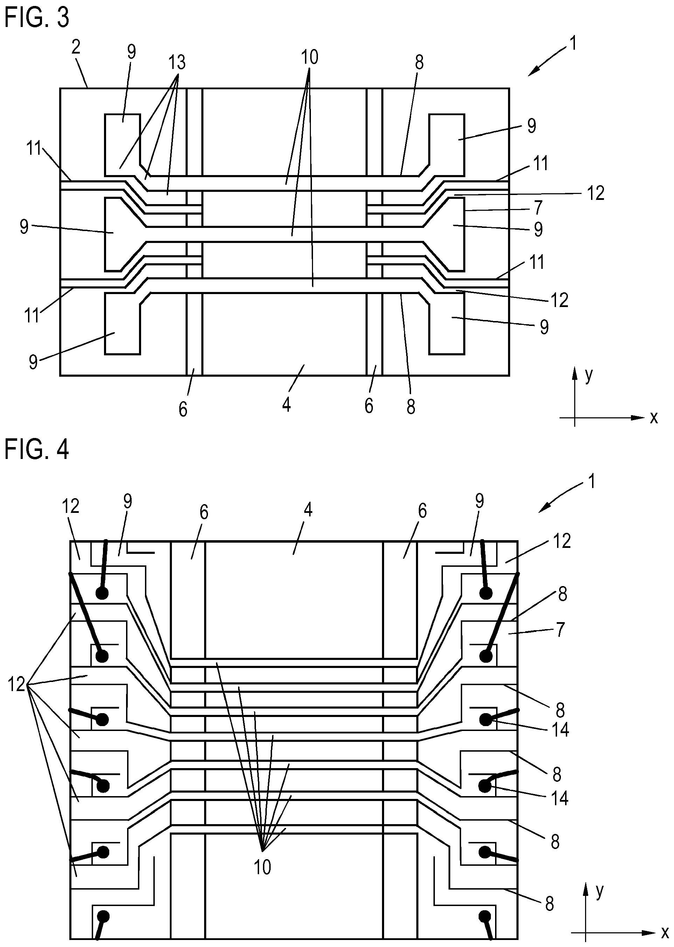

In Fig. 3 ist eine schematische Aufsicht auf ein zweites Ausführungsbeispiel eines erfindungsgemäßen Gassensors 1 dargestellt. In diesem Ausführungsbeispiel sind eine Leiterstruktur 7 sowie zwei Leiterstrukturen 8 vorgesehen, die jeweils einen Stegabschnitt 10 sowie zwei mit diesem Stegabschnitt 10 verbundene Kontaktabschnitte 9 umfassen. Die Kontaktabschnitte 9 der Leiterstrukturen 7, 8 weisen hierbei eine andere Geometrie als in dem vorangehend dargestellten ersten Ausführungsbeispiel auf. Die zwischen den Kontaktabschnitten 9 vorgesehenen Schlitze 11 verlaufen jeweils in dem Bereich 12 der Oberfläche 3 des Substrates 2 zwischen zwei benachbarten Kontaktabschnitten 9. Die Schlitze 11 umfassen mehrere aneinander anschließende gerade Abschnitte 13, die jeweils unter einem Winkel aneinander anschließen. Auf diese Weise wird es ermöglicht, dass die Schlitze 11 zumindest abschnittsweise einer Kontur einer oder beider der Kontaktabschnitte 9 folgen. Die Bereiche 12 der Oberfläche 3 des Substrates 2 zwischen zwei benachbarten Kontaktabschnitten 9 werden von den Schlitzen 11 jeweils mittig durchschnitten.

Auch in diesem Ausführungsbeispiel kann die Leiterstruktur 7 als Heizelement sowie die beiden Leiterstrukturen 8, die zu zwei Seiten in Y-Richtung versetzt neben der Leiterstruktur 7 angeordnet sind, als Widerstandsthermometer eingesetzt werden. Selbstverständlich ist es möglich, dass der Gassensor 1 weitere als Widerstandsthermometer eingesetzte Leiterstrukturen 8 aufweist, die jeweils in unterschiedlichen Abständen entweder in positiver oder negativer Y-Richtung zu der als Heizelement eingesetzten Leiterstruktur 7 versetzt angeordnet werden. Bei weiteren vorgesehenen Leiterstrukturen 8 ergibt sich für deren Stegabschnitte 10 ein größerer Abstand zu dem Stegabschnitt 10 der Leiterstruktur 7. Auf diese Weise kann eine Widerstandsänderung zu verschiedenen, nachfolgenden Zeitpunkten an verschieden weit entfernten Stegabschnitten 10 der Leiterstrukturen 8 gemessen werden.

In Fig. 4 ist eine schematische Aufsicht auf ein drittes Ausführungsbeispiel eines erfindungsgemäßen Gassensors 1 dargestellt. Dieser umfasst eine Leiterstruktur 7, die eine den Graben 4 überspannenden Stegabschnitt 10 sowie zwei Kontaktabschnitte 9 aufweist. In positiver Y-Richtung versetzt ist eine Leiterstruktur 8 angeordnet, die eine den Graben 4 überspannenden Stegabschnitt 10 sowie vier Kontaktabschnitte 9 aufweist, wobei zu beiden Seiten des Grabens 4 jeweils zwei der Kontaktabschnitte 9 angeordnet sind. Entsprechend sind in negativer Y-Richtung von der Leiterstruktur 7 fünf weitere Leiterstrukturen 8 angeordnet, deren Stegabschnitte 10 in unterschiedlichen Abständen zu dem Stegabschnitt 10 der Leiterstruktur 7 angeordnet sind. Auch diese Leiterstrukturen 8 umfassen jeweils vier Kontaktabschnitte 9, von denen jeweils zwei Kontaktabschnitte 9 auf einer Seite des Grabens 4 angeordnet sind. Die Anordnung von jeweils zwei Kontaktabschnitten 9 für jede der Leiterstrukturen 8 zu beiden Seiten des Grabens 4 ermöglicht es, für eine Kalibration des Gassensors 1 an den als Widerstandsthermometer eingesetzten Leiterstrukturen 8 eine Vierpunktmessung zur Bestimmung des elektrischen Widerstandes ihres Stegabschnittes 10 vorzunehmen. Die gezeigten Abstände der Stegabschnitte 10 sind beispielhaft, selbstverständlich ist es auch möglich, andere Abstände zwischen den Stegabschnitten 10 und/oder eine andere Anzahl von Leiterstrukturen 8 zu wählen. In den Bereichen 12 zwischen den Kontaktabschnitten 9 befinden sich ein oder mehrere Schlitze 11, die der Übersichtlichkeit halber nicht gesondert eingezeichnet sind. Bevorzugt befindet sich wenigstens zwischen den Kontaktabschnitten 9 der Leiterstruktur 7 und den benachbarten Kontaktabschnitten 9 der Leiterstrukturen 8 zu beiden Seiten des Grabens 4 jeweils ein Schlitz 11. Weiter bevorzugt kann auch zwischen benachbarten Kontaktabschnitten 9 der Leiterstrukturen 8 jeweils ein Schlitz 11 vorgesehen sein.

In Fig. 4 weiterhin dargestellt sind jeweils mit zwei Kontaktabschnitten 9 der Leiterstrukturen 7, 8 verbundene Bond-Kontakte 14, über die der Stegabschnitt 10 der Leiterstruktur 7 mit einem Strom beaufschlagt werden kann, um diesen zu erwärmen beziehungsweise über die bei den Leiterstrukturen 8 eine Widerstandsveränderung im Stegabschnitt 10 gemessen werden kann. Die Bond-Kontakte der obersten Leiterstruktur 8 sind dabei nicht dargestellt.

Eine Widerstandsmessung der Stegabschnitte 10 der Leiterstrukturen 8 kann beispielsweise dadurch erfolgen, dass die Leiterstrukturen 8 in einer Brückenschaltung verschaltet sind, so dass eine Widerstandsänderung eines Stegabschnittes 10, der den wesentlichen Teil des Widerstandes der jeweiligen Leiterstruktur 8 bildet, in einfacher Weise gemessen werden kann. Dazu kann der Gassensor 1 über die Bond-Kontakte 14 beispielsweise an eine externe Messeinrichtung wie einen Mikrokontroller oder Ähnliches angeschlossen sein.

Eine Messung der Temperaturleitfähigkeit eines die Stegabschnitte 10 der Leiterstrukturen 7, 8 umgebenden Gases oder Gasgemischs mittels eines Ausführungsbeispiels eines erfindungsgemäßen Verfahrens kann derart erfolgen, dass der Stegabschnitt 10 der Leiterstruktur 7 durch einen Strompuls erwärmt wird, wobei an den Stegabschnitten 10 der Leiterstrukturen 8 jeweils eine zeitabhängige Widerstandsmessung vorgenommen wird. Abhängig von der Temperaturleitfähigkeit des Gases sowie von dem Abstand des jeweiligen Stegabschnittes 10 der Leiterstruktur 8 von dem Stegabschnitt 10 der Leiterstruktur 7 ergeben sich unterschiedliche Zeitpunkte, zu denen die von dem erwärmten Stegabschnitt 10 der Leiterstruktur 7 ausgehende Wärme eine Widerstandsveränderung der Stegabschnitte 10 der Leiterstrukturen 8 hervorruft. Bei bekannten Abständen der Stegabschnitte 10 zueinander kann aus den zeitabhängigen Messungen auf die Temperaturleitfähigkeit des Gases bzw. des Gasgemisches geschlossen werden. Die Temperaturleitfähigkeit kann beispielsweise dazu herangezogen werden, um den Typ des Gases und/oder die Art der Zusammensetzung des Gasgemisches zu bestimmen.

Eine Messung der Wärmeleitfähigkeit eines die Stegabschnitte 10 der Leiterstrukturen 7, 8 umgebenden Gases oder Gasgemischs mittels eines Ausführungsbeispiels eines erfindungsgemäßen Verfahrens kann derart erfolgen, dass der Stegabschnitt 10 der Leiterstruktur 7 durch einen Strompuls erwärmt wird, wobei an den Stegabschnitten 10 der Leiterstrukturen 8 jeweils ein Temperaturwert gemessen wird. Abhängig von der Wärmeleitfähigkeit des Gases sowie von dem Abstand des jeweiligen Stegabschnittes 10 der Leiterstruktur 8 von dem Stegabschnitt 10 der Leiterstruktur 7 ergeben sich unterschiedliche Temperaturen, auf welche die Stegabschnitte 10 der Leiterstrukturen 8 durch die von dem erwärmten Stegabschnitt 10 der Leiterstruktur 7 ausgehende Wärme erwärmt werden. Die Temperatur der Stegabschnitte 10 der Leiterstrukturen 8 kann als eine Widerstandsveränderung der Stegabschnitte 10 der Leiterstrukturen 8 gemessen werden.

Aus den Temperaturwerten, welche an den Leiterstrukturen 8 gemessen werden, können anschließend Parameter berechnet werden, die eine Ausgleichsgerade parametrisieren, welche den Zusammenhang zwischen dem Logarithmus der Entfernung des jeweiligen Stegabschnitts 10 einer der Leiterstrukturen 8 von dem Stegabschnitt 10 der geheizten Leiterstruktur 7 und dem Temperaturmesswert beschreibt. Aus diesen Parametern kann anschließend die Wärmeleitfähigkeit des die Stegabschnitte 10 umgebenden Gases oder Gasgemischs bestimmt werden. Durch die Verwendung von Leiterstrukturen 8, welche zu beiden Seiten entlang des Grabens 4 von der Leiterstruktur 7 beabstandet angeordnet sind, können Temperaturwerte für unterschiedliche Entfernungen und/oder redundante Temperaturwerte für gleiche Entfernungen erhalten werden.

### Bezugszeichenliste

- 1: Gassensor
- 2: Substrat
- 3: Oberfläche
- 4: Graben
- 5: Boden
- 6: Wände
- 7: Leiterstruktur
- 8: Leiterstruktur
- 9: Kontaktabschnitt
- 10: Stegabschnitt
- 11: Schlitz
- 12: Bereich
- 13: Abschnitt
- 14: Bond-Kontakt

## Patentansprüche

1. Thermischer Gassensor zur Messung der Temperaturleitfähigkeit und/oder der Wärmeleitfähigkeit eines Gases oder Gasgemisches, umfassend ein Substrat (2), in dessen Oberfläche (3) ein Graben (4) ausgebildet ist, sowie wenigstens zwei beabstandet voneinander auf der Oberfläche (3) des Substrates (4) angeordnete Leiterstrukturen (7, 8), wobei die Leiterstrukturen (7, 8) jeweils wenigstens zwei Kontaktabschnitte (9) sowie einen mit diesen Kontaktabschnitten (9) verbundenen Stegabschnitt (10) umfassen, wobei die Stegabschnitte (10) der Leiterstrukturen (7, 8) beabstandet voneinander den Graben (4) überspannen, **dadurch gekennzeichnet, dass** in wenigstens einem Bereich der Oberfläche (3) des Substrates (2) zwischen wenigstens zwei Kontaktabschnitten (9) unterschiedlicher Leiterstrukturen (7, 8) wenigstens ein Schlitz (11) ausgebildet ist.

2. Thermischer Gassensor nach Anspruch 1, **dadurch gekennzeichnet, dass** sich der Graben (4) entlang einer Längsrichtung erstreckt, wobei die Stegabschnitte (10) der Leiterstrukturen (7, 8) den Graben (4) parallel oder im Wesentlichen parallel in einer Querrichtung überspannen.

3. Thermischer Gassensor nach Anspruch 1 oder 2, **dadurch gekennzeichnet, dass** der Graben (4) einen Boden (5), der insbesondere parallel oder im Wesentlichen parallel zur Oberfläche (3) des Substrates (2) ist, sowie wenigstens zwei Wände (6) umfasst, wobei die Wände (6) zwischen der Oberfläche (3) des Substrates (2) und dem Boden (5) des Grabens (4) verlaufen, wobei sich der wenigstens eine Schlitz (11) wenigstens von der Oberseite (3) des Substrates (2) zum Boden (5) des Grabens (4) erstreckt und/oder wobei der wenigstens eine Schlitz (11) durch eine Wand (6) des Grabens (4) verläuft.

4. Thermischer Gassensor nach einem der vorangehenden Ansprüche, **dadurch gekennzeichnet, dass** der Schlitz (11) einen oder mehrere aneinander anschließende gerade Abschnitte (13) umfasst, wobei der Schlitz (11) den Bereich der Oberfläche (3) des Substrates (2) zwischen den wenigstens zwei Kontaktabschnitten (9) ganz oder teilweise durchschneidet.

5. Thermischer Gassensor nach einem der vorangehenden Ansprüche, **dadurch gekennzeichnet, dass** der Graben (4) und/oder der wenigstens eine Schlitz (11) durch ein bereichsweises Ätzen des Substrates (2) gefertigt ist.

6. Thermischer Gassensor nach einem der vorangehenden Ansprüche, **dadurch gekennzeichnet, dass** der Schlitz (11) eine Breite zwischen 1 µm und 50 µm, insbesondere zwischen 10 µm und 20 µm, aufweist und/oder dass der Graben (4) eine Breite zwischen 0,5 mm und 5 mm und/oder eine Tiefe zwischen 100 µm und 500 µm aufweist.

7. Thermischer Gassensor nach einem der vorangehenden Ansprüche, **dadurch gekennzeichnet, dass** die Leiterstrukturen (7, 8) über die wenigstens zwei Kontaktabschnitte (9) zur Bestromung des Stegabschnitts (10) der Leiterstruktur (7, 8) bestrombar und/oder zur Messung eines Widerstandes des Stegabschnitts (10) kontaktierbar sind, wobei wenigstens eine der Leiterstrukturen (7, 8) zwei weitere Kontaktabschnitte (9) zur Messung eines Potentialabfalls über dem Stegabschnitt (10) aufweisen.

8. Thermischer Gassensor nach Anspruch 7, **dadurch gekennzeichnet, dass** auf der Oberfläche (3) des Substrates (2) zu zwei gegenüberliegenden Seiten einer ein Heizelement bildenden Leiterstruktur (7, 8) mit zwei Kontaktabschnitten (9) jeweils eine ein Widerstandsthermometer bildende Leiterstruktur (7, 8) mit vier Kontaktabschnitten (9) angeordnet ist, wobei zwischen den Kontaktabschnitten (9) des Heizelements und jeweils wenigstens einem Kontaktabschnitt (9) der Widerstandsthermometer ein Schlitz (11) ausgebildet ist.

9. Thermischer Gassensor nach einem der vorangehenden Ansprüche, **dadurch gekennzeichnet, dass** die Leiterstrukturen (7, 8) zumindest teilweise aus Nickel bestehen und/oder dass das Substrat (2) zumindest teilweise aus Silizium und/oder Siliziumnitrid besteht.

10. Verfahren zur Messung der Temperaturleitfähigkeit eines Gases oder des Gasgemischs mittels eines thermischen Gassensors (1) nach einem der vorangehenden Ansprüche,
umfassend die Schritte:
- Bereitstellen des thermischen Gassensors (1) sowie des Gases oder Gasgemischs,
- Anlegen eines zeitlich begrenzten Strompulses an eine Leiterstruktur (7, 8),
- Zeitabhängige Messung des elektrischen Widerstandes wenigstens einer weiteren Leiterstruktur (7, 8),
- Bestimmung der Temperaturleitfähigkeit aus der zeitabhängigen Messung des elektrischen Widerstandes der wenigstens einen weiteren Leiterstruktur (7, 8) und dem Abstand der Stegabschnitte (10) der Leiterstrukturen (7, 8) zueinander.

11. Verfahren zur Messung der Wärmeleitfähigkeit eines Gases oder des Gasgemischs mittels eines thermischen Gassensors (1) nach einem der vorangehenden Ansprüche, wobei der Gassensor wenigstens drei beabstandet voneinander auf der Oberfläche (3) des Substrates (4) angeordnete Leiterstrukturen (7, 8) umfasst, umfassend die Schritte:
- Bereitstellen des thermischen Gassensors (1) sowie des Gases oder Gasgemischs,
- Anlegen eines zeitlich begrenzten Strompulses an eine Leiterstruktur (7, 8) zum Heizen dieser Leiterstruktur (7, 8),
- Messung von Temperaturwerten mittels der wenigstens zwei weiteren Leiterstrukturen (7, 8),
- Bestimmung der Wärmeleitfähigkeit aus Temperaturwerten der wenigstens zwei weiteren Leiterstrukturen (7, 8) sowie dem Abstand der Stegabschnitte (10) der zwei weiteren Leiterstrukturen (7, 8) zu dem Stegabschnitt (10) der geheizten Leiterstruktur (7, 8).
